# EUROPEAN PATENT SPECIFICATION

(11) **EP 1 971 566 B1**
(45) Date of publication and mention of the grant of the patent: **12.11.2014**
(21) Application number: 05849575.5
(22) Date of filing: 09.12.2005
(51) Int. Cl.: C07C 51/43, C07C 51/265

(54) **A PROCESS FOR PREPARING HIGH PURITY TEREPHTHALIC ACID**
VERFAHREN ZUR HERSTELLUNG HOCHREINER TEREPHTHALSÄURE
PROCEDE POUR PREPARER DE L'ACIDE TEREPHTALIQUE A PURETE ELEVEE

(43) Date of publication of application: 24.09.2008
(73) Proprietor: M&G USA Corporation, Apple Grove, WV 25502 (US)
(72) Inventor: HRONEC, Milan, 821 05 Bratislava (SK); AL GHATTA, Hussain, I-03014 Fiuggi (FR) (IT); RUGGIERI, Roberto, I-20123 Milano (IT)
(74) Representative: Gerbino, Angelo
(86) International application number: PCT/IT2005/000726
(87) International publication number: WO 2007/066365

(56) References cited:
- US-A- 3 299 125
- US-A- 6 034 269
- US-B1- 6 175 038
- US-B1- 6 307 099
- US-B1- 6 565 754

## Description

### Background

Pure terephthalic acid (PTA), an important raw material used in the production of poly(ethylene terephthalate) (PET) for conversion into fibers, films and containers, is commercially produced by purifying crude-or technical-grade terephthalic acid produced by catalytic, liquid phase air oxidation of p-xylene (PX). Practically all technical-grade PTA is produced by catalytic, liquid phase air oxidation of p-xylene.

Commercial processes use acetic acid as a solvent and a multivalent heavy metal catalyst, most widely based on cobalt and manganese compounds, and a promoter, with bromine or bromide ions as the renewable source of free radicals.

Acetic acid, air, p-xylene and catalyst are fed continuously into an oxidation reactor that is maintained at from 175°C to 225°C under pressure of 1.5-3.0 MPa. The feed weight ratio of acetic acid to p-xylene is typically less than 5:1. Air is added in amounts in excess of stoichiometric requirements to minimize formation of by-products. The oxidation reaction is exothermic, and heat is typically removed by allowing the acetic acid solvent to boil. The corresponding vapor is condensed and most of the condensate is refluxed to the reactor. The residence time is typically 30 minutes to 2 hours, depending on the process.

The effluent from the reactor is a slurry of crude terephthalic acid crystals which are recovered by filtration, washed, dried and conveyed to storage. The crystals are thereafter fed to a separate purification steps (See United States Patent No. 5,350,133). While the main impurity is 4-carboxybenzaldehyde (4-CBA), p-toluic acid (pTA) is also present in relevant amount. Although the purity of crude-grade PTA is typically greater than 99%, it is not pure enough for the PET made from it to reach the required degree of polymerization.

From United States Patent No. 6,034,269, a process is known for production of high purity terephthalic acid by catalytic, liquid phase oxidation of p-xylene carried out in a plug flow reaction zone, wherein a high weight ratio between the solvent (acetic acid) and p-xylene and temperature and pressure sufficient to maintain PTA in solution as it is formed are used.

The acid is crystallized from the resulting reaction medium and recovered without the need for separate purification. While the purity can be as high as 99.95%, pTA is present in amount higher than 80-90 ppm.

In United States Patent No. 6,307,099 a process for homogeneous liquid phase oxidation of p-xylene to PTA is described wherein the 4-CBA content of the recovered terephthalic acid is most preferably no more than about 500 ppm, e.g., 20 to 300 ppm. No data concerning pTA, the color parameters and catalyst residues are reported. The process requires one to circulate a large volume of the solvent and uses a very high concentration of oxidation catalyst, calculated to the oxidized p-xylene. The process is operated under reaction conditions, where substantially all of the terephthalic acid produced in the oxidation reaction remains in the solution during the reaction. The detailed description of this patent specifies, the possibility of some precipitation during the reaction, e.g. up to 10 % but desirably no more than about 2% by weight of the terephthalic acid produced may precipitate during the course of the reaction. The patents (United States Patent Numbers 6,034,269 and United States Patent Numbers 6,037,099) do not specify the oxidation catalyst, its concentration, and its significant influence on the quality of terephthalic acid. Under these patents the selection of the catalyst and oxidation promoter is within conventional practice.

US-6 175 038 B1 discloses a process for the oxidation of para-xylene to terephthalic acid by using (see Example 2) a catalyst wherein the weight ratio Co:Zr is 1:1 and the weight ratio Co/Mn is 1:2, as well as a reaction mixture containing 15% by weight of p-xylene and 77.5% by weight of acetic acid implying an acetic acid/ para-xylene weight ratio of about 5.

### Summary of the invention

The present invention relates to a process for preparing highly pure terephthalic acid comprising the steps of
A) oxidizing para-xylene to terephthalic acid with air in the presence of a liquid reaction phase maintained at a temperature between 180°C and 230°C,
   wherein the liquid reaction phase comprises para- xylene, acetic acid, water, and a catalyst composition, wherein the water is 5 to 12 percent by weight of the acetic acid,
   the acetic acid/p-xylene weight ratio is not less than 30:1 and such that 12 to 50% of the reacted terephthalic acid is present as a solid at the oxidation temperature, and the catalyst composition comprises Cobalt, Manganese, and Bromine in combination with at least one element selected from the group consisting of Zirconium and Hafnium wherein the atomic ratio of Co: Mn: Br: is in the range of 1:0.2 - 1.0:1.1 - 2.7 and the atomic ratio of Cobalt to the elements selected from the group consist-ing of Zirconium and Hafnium is 1:0.03 - 0.3, wherein the total weight of Co and Mn is 100-500mg per 1 kg of the liquid reaction phase; and
B) recovering the terephthalic acid by crystallization at a temperature in the range from 150°C to 80°C.

### Detailed Description

It has now unexpectedly been found that it is possible to produce by catalytic, liquid phase oxidation of p-xylene a highly pure PTA without the purification step. The process described herein produces, without a purification step, a PTA which contains small amounts of impurities, preferably less than 40 ppm by weight in total of 4-CBA and pTA, and less than 20 ppm of pTA, and having excellent color parameters.

Such very high quality of PTA can be obtained under very specific reaction and concentration conditions disclosed in this specification. The catalytic, liquid phase oxidation of p-xylene must be conducted in acetic acid as solvent, at temperature, pressure and with acetic acid to p-xylene weight ratio such that 12 to 50 % of the formed PTA is not maintained in solution, and operating under specific temperature conditions in presence of very specific composition and concentration of a catalyst. The catalyst must contain compounds that comprise Co and Mn with Zr and/or Hf compounds, and, as a promoter, bromine and/or bromine compounds; wherein the atomic ratio Co: Mn: Br is in the range of 1:0.2-1.0:1.1-2.7, preferably 1:0.3-0.8:1.1-1.8 and the atomic ratio of Co:Zr and/or Hf is 1:0.03-0.3.

The oxidation is conducted in a stirred reactor under stir-ring conditions suitable to homogenize the liquid phase and provide similar temperature conditions in all points of the reactor, at a temperature comprised in the range from 195°C to 220°C or, preferably, according to a temperature profile starting from 180° to 200°C and then up to 230°C. Preferably, the temperature in the last period is of 205°-215°C. Temperatures higher than 230°C are not recommended because acetic acid reacts and is lost. However, in some cases it is convenient after finishing the oxidation reaction to heat the reaction mixture for a short time to the temperature 230-240°C in the absence of an air flow (e.g. for 10 - 30 min).

The concentration of the catalyst, expressed as total weight of Co and Mn per 1 kg of liquid reaction phase, is of 100-500 mg.

PTA is recovered from the reaction phase by crystallization at temperatures from 80° to 150°C, and then washed with acetic acid and/or water. The mother liquor is in part recycled to the oxidation reactor and in part regenerated.

The color L* determined according to the CIE Standard method on pulverized PTA having average particle size of less than 50 *µ*m has a minimum of 95.5.

The impurities derived from the catalyst expressed as Co and Mn metal were in total less than 8 ppm by weight; in particular Co was less than 4 ppm and Mn less than 2 ppm.

PTA crystallized from the reaction liquor, at least in part, is in the form of crystals distinctly angular, e.g. having a rhomboid structure and thereby different from the PTA produced according to the commonly used prior art processes of catalytic heterogeneous liquid phase oxidation of p-xylene, wherein the crystals tend to be rounded agglomerates of smaller crystals.

The acetic acid used as the solvent contains from 1 to 15 wt% water, preferably 5 to 10%.

The acetic acid / p-xylene weight ratio is not less than 30:1, but must be such that the 12 to 50%, particularly 20-30 wt% of the PTA after the oxidation reaction is present as solid phase.

The oxidation reaction is exothermic. Typically in the known processes the heat has been removed by allowing the acetic acid solvent to boil, with the resulting vapor being condensed and the condensate, in varying amount, refluxed to the reactor. Typically, in the process of the present invention the reaction temperature and pressure are maintained at the level necessary to maintain the preferred temperature profile and to reach the preferred temperature of the last heating step.

The reaction can also be conducted in a plug flow reactor.

In the plug flow reactor, molecular oxygen is dissolved in the feed stream to achieve a concentration of dissolved oxygen in excess on the stoichiometric value. Using a stirred reactor, air is passed through the liquid phase with a flow rate sufficient to remove heat and to not exceed the inflammability limit in the top of the reactor. The source of oxygen can be pure oxygen, air, or any convenient oxygen-containing gas.

Examples of cobalt, manganese, zirconium and hafnium compounds usable as catalyst component are the acetates, carbonates, hydroxides and oxides. Examples of bromine or bromine containing compounds are bromine, HBr, NaBr, KBr, and organic bromides which are known to provide bromide ions at the temperature of oxidation, such as bromobenzenes, benzylbromide and tetrabromoethane.

The reaction time in a stirred reactor depends on the reaction conditions and is generally from 13 to 45 minutes.

The PTA obtained with the process of the present invention, thanks to its very low content of both 4-CBA and pTA, is particularly suitable for the production of high molecular weight polyalkylene terephthalates and copolymers thereof.

The following examples are given to illustrate and not to limit the scope of the present invention.

### EXAMPLES

### Oxidation conditions

Oxidation of p-xylene with air is carried out in a 250 ml reactor of a titanium alloy. The reactor is fitted with a magnetic stirred system operating at 100-3000 rpm, has an air inlet at the bottom and an outlet through a condenser equipped with a phase separator, pressure and temperature regulator, electric heating mantle and outlet for a products probe.

The reactor is charged with p-xylene, catalyst and solvent (acetic acid added with water) and pressurized with nitrogen to 2.5 MPa. The temperature is then raised to the desired temperature over 15-20 minutes. When the temperature of the liquid medium inside the reactor reaches the desired temperature, the flow of air into the liquid phase is established at 0.5 l/min. and the stirred speed at 2200 rpm. The outlet gas is continuously monitored using an oxygen analyzer. When the oxygen consumption stops, the stirrer speed is lowered to 200-300 rpm and the heating of the reactor is terminated. The temperature of the reactor contents is cooled to approximately 85°C in 10-60 min.

During this cooling period, PTA crystallizes from the stirred liquid medium. The solid is isolated at about 80°C, washed (3 times with 10 ml acetic acid and 1 time with 20 ml H₂O), dried at 80°C for 5h, weighed, pulverized and analyzed. The content of 4-CBA and pTA is determined by HPLC.

### Analytical Determinations

pTA and 4-CBA are measured by liquid chromatography (HPLC) using a Du Pont "Zorbax" NH₂ column, an ammonium phosphate buffer solution (pH adjusted to 4.25 with concentrated NH₄OH if it has to be raised, with H₃PO₄ if it has to be lowered) and a 254 nm absorbance detector. A sample of dry PTA of 0.2 + 0.0005 g is dissolved with 20 ml of a 3.7 wt% ammonium hydroxide solution; 20 ml of distilled water and 10 ml benzyl alcohol are added to the PTA sample.

The pH is adjusted to 7 (6.8-7.2) with concentrated H₃PO₄.

Before use, the column is flushed initially with CH₃CN, and after 15 minutes with distilled water. The buffer solution is run through the column for 16 h at a rate of 1.3 ml/min to stabilize the column.

A Perkin-Elmer Sigma 10 data processor and Spectra-Physics computing integrator (or equivalents) are used for analysis.

### Example 1

The reactor was charged with 2.5 g p-xylene, 150 g acetic acid containing 10 wt% H₂O, 0.100 g Co(CH₃COO)₂.4H₂O and Mn (II) acetate, Zr (IV) acetate, HBr (47% solution in H₂O) in the atomic ratio Co:Mn:Zr:Br = 1:0.6:0.05:1.7. The mixture was oxidized with air (flow 0.5 l/min) at 195°C for 20 minutes, then during 3 min the temperature was increased to 210°C and at this temperature oxidation proceeded for 22 min to reach complete conversion of p-xylene. The HPLC analysis of terephthalic acid determined that it contained 11 ppm of 4-CBA and 4 ppm of p-toluic acid. The color parameter L* was 95.8.

### Example 2

The procedure of Example 1 was repeated except that hafnium was employed instead of zirconium in the amount corresponding to the atomic ratio Co:Mn:Hf:Br = 1:0.6:0.1:1.7. The terephthalic acid contained 14 ppm of 4-CBA and 5 ppm of p-toluic acid.

### Example 3 (Comparative)

The procedure of Example 1 was repeated except that no zirconium was added. The quality of produced terephthalic acid in 4-CBA and 6 ppm of p-toluic acid.

### Examples 4 to 7

The procedure of Example 1 was repeated except that the mixture was oxidized with air at 195 °C for 15 min and at 210 °C for 7 min. The composition of the catalysts in the experiments was changed as illustrated in Table 1.

**Table 1**

| Exp. No | Atomic ratio Co:Mn:Zr:Br | Purity of terephthalic acid, ppm | |
|---|---|---|---|
| | | 4-CBA | p-toluic acid |
| 4 comparative | 1:1.2:0.1:2.3 | 85 | 9 |
| 5 | 1:0.8:0.1:2.3 | 20 | 5 |
| 6 | 1:0.2:0.1:2.3 | 14 | 4 |
| 7 comparative | 1:0:0.1:2.3 | low-rate, | no-PTA |

The results unambiguously demonstrate the very high influence of the catalyst composition on the purity of the formed terephthalic acid. A very high purity of PTA is reached only if the concentration of manganese varies in a certain range. In the absence of manganese (Exp. No. 7) the rate of oxidation is very low and PTA is practically not formed.

### Examples 8 to 11

The procedure of example 4 was repeated except that the composition of the catalysts in the experiment was changed as is illustrated in Table 2

**Table 2**

| Exp. No | Atomic ratio Co:Mn:Zr:Br | Purity of PTA, ppm | |
|---|---|---|---|
| | | 4-CBA | p-toluic acid |
| 8 comparative | 1:1.2:0:2.3 | 90 | 5 |
| 4 comparative | 1:1.2:0.1:2.3 | 85 | 9 |
| 9 comparative | 1:0.6:0:2.3 | 104 | 4 |
| 10 | 1:0.6:0.1:2.3 | 6 | 4 |
| 11 comparative | 1:0.2:0:2.3 | 118 | 8 |
| 6 | 1:0.2:0.1:2.3 | 14 | 4 |

These results clearly show that the synergistic effect of zirconium is strongly influenced by the atomic ratio of Co:Mn in the oxidation catalysts. When the ratio of Co:Mn is 1:1.2, the synergistic effect of zirconium on the purity of terephthalic acid is negligible.

### Example 12

The reactor was charged with 2.5 g p-xylene, 150 g acetic acid containing 5 wt% H₂O, 0.120 g cobalt acetate tetrahydrate and Mn(II)acetate, Zr(IV)acetate, HBr (47% solution in H₂O) in the atomic ratio Co:Mn:Zr:Br = 1: 0.2:0.1:1.35. The flow of air used for oxidation of p-xylene was during the oxidation changed in the range 1.5 to 0.05 l/min depending on the oxygen content in the off-gas. The mixture was oxidized at 195°C for 7 min, then during 2 min the temperature was increased to 205°C and at this temperature oxidation proceeded for 9 min (total reaction time 18 min). The obtained terephthalic acid contained 24 ppm of 4-CBA and 6 ppm of p-toluic acid.

The experiment demonstrates that at specific reaction conditions and composition of the catalyst it is possible to obtain high purity PTA at the temperature 205 °C in the last stage of oxidation

### Example 13

The procedure of Example 12 was repeated except that oxidation of p-xylene was carried out isothermally at 205°C for 18 min. The terephthalic acid contained 42 ppm of 4-CBA and 6 ppm of p-toluic acid. The color parameter L* is 93.1.

The comparison of results of experiments 12 and 13 confirms that isothermal oxidation of p-xylene produces terephthalic acid with lower purity than step-wise oxidation at different temperatures.

### Examples 14 to 17

The procedure of Example 4 was repeated except that the composition of the catalysts in experiments was Co:Mn:Zr = 1:0.6:0.1 and the atomic ratio Co:Br was changed as is described in Table 3.

**Table 3**

| Exp. no | Atomic ratio Co:Br | Purity of PTA, ppm | |
|---|---|---|---|
| | | 4-CBA | p-toluic acid |
| 14 | 1:1.00 | 84 | 4 |
| 15 | 1:1.35 | 22 | 4 |
| 16 | 1:2.70 | 32 | 5 |
| 17 | 1:3.44 | 52 | 6 |

As is seen from the results, the concentration of bromine in the reaction system must be optimal in order to obtain high purity PTA.

### Example 18

The reactor was charged with 5.0 g p-xylene, 150 g acetic acid containing 5 wt% water, 0.20 g cobalt acetate tetrahydrate and Mn(II)acetate, Zr(IV)acetate, HBr (47% solution in H₂O) in the atomic ratio Co:Mn:Zr:Br = 1:0.6:0.1:2.3. The flow of air was during oxidation changed in the range 1.5 to 0.05 l/min depending on the oxygen content in the off-gas. The mixture was oxidized at 190°C for 10 min, then during 5 min the temperature increased to 220°C and at this temperature oxidation proceeded for 5 min. In the next step the inlet of air flow was stopped and during 8 min the reaction mixture is heated to 235 °C and at this temperature stirred for 20 min. The formed terephthalic acid contained 33 ppm of 4-CBA and 5 ppm of p-toluic acid.

### Example 19

The procedure of Example 18 was repeated except that the reaction mixture after the oxidation reaction was not heated to 235°C. The formed terephthalic acid contains 117 ppm of 4-CBA and 27 ppm of p-toluic acid. The results of Examples 18 and 19 confirm that the subsequent heating of the reaction mixture in the absence of air increases its purity.

## Claims

1. A process for preparing highly pure terephthalic acid comprising the steps of
A) oxidizing para-xylene to terephthalic acid with air in the presence of a liquid reaction phase maintained at a temperature between 180°C and 230°C,
wherein the liquid reaction phase comprises para- xylene, acetic acid, water, and a catalyst composition, wherein the water is 5 to 12 percent by weight of the acetic acid,
the acetic acid/p-xylene weight ratio is not less than 30:1 and such that 12 to 50% of the reacted terephthalic acid is present as a solid at the oxidation temperature, and
the catalyst composition comprises Cobalt, Manganese, and Bromine in combination with at least one element selected from the group consisting of Zirconium and Hafnium wherein the atomic ratio of Co: Mn: Br: is in the range of 1:0.2 - 1.0:1.1 - 2.7 and the atomic ratio of Cobalt to the elements selected from the group consist-ing of Zirconium and Hafnium is 1:0.03 - 0.3, wherein the total weight of Co and Mn is 100- 500 mg per 1 kg of the liquid reaction phase; and
B) recovering the terephthalic acid by crystallization at a temperature in the range from 150°C to 80°C.

2. The process according to claim 1 wherein the oxidation temperature is in the range of 180°C to 200°C in a first oxidation stage and is in the range from 200°C to 225°C in a last oxidation stage, while the degree of conversion of p-xylene to acid derivatives in the first oxidation stage is within the range from 50 to 80 percent.

3. The process according to claim 2, wherein after finishing the oxidizing step and prior to the recovering step, the reaction mixture is heated for 10-30 min in the temperature range of 230 - 240 °C in the absence of the air.

## Patentansprüche

1. Verfahren zur Herstellung hoch reiner Terephthalsäure, umfassend die Schritte
A) Oxidieren von para-Xylen zu Terephthalsäure mit Luft in Gegenwart einer flüssigen Reaktionsphase, die bei einer Temperatur zwischen 180°C und 230°C gehalten wird,
wobei die flüssige Reaktionsphase para-Xylen, Essigsäure, Wasser und eine Katalysatorzusammensetzung umfasst, wobei das Wasser 5 bis 12 Gewichtsprozent der Essigsäure entspricht,
wobei das Essigsäure/p-Xylen-Gewichtsverhältnis nicht weniger als 30:1 ist und so, dass 12 bis 50% der umgesetzten Terephthalsäure als ein Feststoff bei der Oxidationstemperatur vorliegt, und
wobei die Katalysatorzusammensetzung Cobalt, Mangan und Brom in Kombination mit mindestens einem Element ausgewählt aus der Gruppe bestehend aus Zirkonium und Hafnium umfasst, wobei das Atomverhältnis von Co: Mn: Br im Bereich von 1:0,2 - 1,0:1,1 - 2,7 ist und das Atomverhältnis von Cobalt zu den Elementen ausgewählt aus der Gruppe bestehend aus Zirkonium und Hafnium 1:0,03 - 0,3 ist, wobei das Gesamtgewicht von Co und Mn 100-500 mg pro 1 kg der flüssigen Reaktionsphase ist; und
B) Rückgewinnen der Terephthalsäure durch Kristallisation bei einer Temperatur im Bereich von 150°C bis 80°C.

2. Verfahren nach Anspruch 1, wobei die Oxidationstemperatur im Bereich von 180°C bis 200°C in einer ersten Oxidationsstufe liegt und im Bereich von 200°C bis 225°C in einer letzten Oxidationsstufe liegt, während der Grad der Umsetzung von p-Xylen zu Säurederivaten in der ersten Oxidationsstufe innerhalb des Bereichs von 50 bis 80 Prozent liegt.

3. Verfahren nach Anspruch 2, wobei nach Beendigung des Oxidationsschritts und vor dem Rückgewinnungsschritt die Reaktionsmischung für 10-30 min im Temperaturbereich von 230-240°C in Abwesenheit der Luft erhitzt wird.

## Revendications

1. Procédé pour préparer de l'acide téréphtalique très pur comprenant les étapes de :
A) oxydation du para-xylène en acide téréphtalique avec de l'air en présence d'une phase réactionnelle liquide maintenue à une température entre 180°C et 230°C,
où la phase réactionnelle liquide comprend du para-xylène, de l'acide acétique, de l'eau et une composition catalytique, où l'eau est 5 à 12 pourcent en poids de l'acide acétique, le rapport en poids acide acétique/p-xylène n'est pas inférieur à 30:1 et tel que 12 à 50 % de l'acide téréphtalique qui a réagi sont présents sous forme d'un solide à la température d'oxydation, et la composition catalytique comprend du cobalt, du manganèse et du brome en combinaison avec au moins un élément choisi dans le groupe consistant en le zirconium et le hafnium où le rapport atomique de Co : Mn : Br : est dans la plage de 1:0,2 - 1,0:1,1 - 2,7 et le rapport atomique du cobalt aux éléments choisis dans le groupe consistant en le zirconium et le hafnium est 1:0,03 - 0,3, où le poids total de Co et Mn est 100 - 500 mg par 1 kg de phase réactionnelle liquide ; et
B) récupération de l'acide téréphtalique par cristallisation à une température dans la plage de 150°C à 80°C.

2. Procédé selon la revendication 1 où la température d'oxydation est dans la plage de 180°C à 200°C dans un premier stade d'oxydation et est dans la plage de 200°C à 225°C dans un dernier stade d'oxydation, tandis que le degré de conversion du p-xylène en dérivés acides dans le premier stade d'oxydation est dans la plage de 50 à 80 pourcent.

3. Procédé selon la revendication 2, où après la fin de l'étape d'oxydation et avant l'étape de récupération, le mélange réactionnel est chauffé pendant 10 - 30 min dans la plage de température de 230 - 240°C en l'absence d'air.
